# EUROPÄISCHE PATENTANMELDUNG

(11) **EP 0 784 053 A1**
(43) Veröffentlichungstag der Anmeldung: **16.07.1997**
(21) Anmeldenummer: 97100080.7
(22) Anmeldetag: 04.01.1997
(51) Int. Cl.: C07D 249/12, C07C 243/22, C07C 243/28, C07D 471/04

(54) **Verfahren zur Herstellung von Triazolinonherbiziden**

(30) Priorität: 15.01.1996 DE 19601190
(71) Anmelder: Degussa Aktiengesellschaft, 60311 Frankfurt (DE)
(72) Erfinder: Weckbecker, Christoph, Dr., 63457 Hanau (DE); Drauz, Karlheinz, Prof.-Dr., 63579 Freigericht (DE)

(57) **Zusammenfassung**

Verfahren zur Herstellung von substituierten 1,2,4-Triazolin-3(2H)-onen der allgemeinen Formel (I) 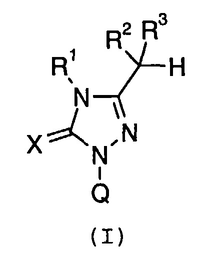 durch Umsetzung eines Amides der allgemeinen Formel (IV) 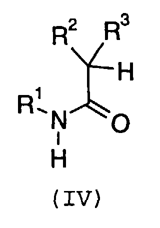 mit Phosgen oder Thiophosgen bzw. einem Phosgenersatz oder Thiophosgenersatz zu einer Verbindung der allgemeinen Formel V 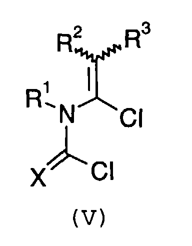 und anschließender Reaktion von (V) mit einer Verbindung der allgemeinen Formel (VI) oder eines Säureadditionssalzes von (VI)

Q―NH-NH-CO-R²⁶ (VI)

zu Verbindungen der allgemeinen Formel (I).

Verbindungen der allgemeinen Formel (I) können als Herbizide verwendet werden.

## Beschreibung

Die Erfindung betrifft ein neues Verfahren zur Herstellung von 1,2,4-Triazolin-3(2H)-onen, in Folge auch vereinfacht als Triazolinone bezeichnet, eine Verbindungsklasse der allgemeinen Formel (I), 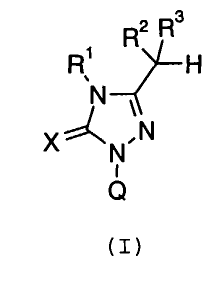 worin
- X =: O oder S,
- R¹ =: (C₂-C₈) Alkoxyalkyl, (C₂-C₈) Haloalkoxyalkyl, (C₁-C₈) Alkyl, (C₁-C₈) Haloalkyl, (C₁-C₈) Cyanoalkyl, (C₂-C₈) Alkylthioalkyl, (C₂-C₈) Alkylsulfinylalkyl, (C₂-C₈) Alkylsulfonylalkyl, (C₇-C₈) Arylalkyl wie Benzyl, (C₂-C₈) Alkenyl, (C₂-C₈) Haloalkenyl, (C₂-C₈) Alkinyl, (C₂-C₈) Haloalkinyl, Aryl wie Naphthyl oder Phenyl, das ein- oder mehrfach mit Halogen, (C₁-C₃) Alkyl oder (C₁-C₃) Alkoxy substituiert sein kann, Heteroaryl wie Pyridin, wobei Heteroaryl ggf. mit Halogen substituiert sein kann, bedeuten,
- R² und R³,: unabhängig voneinander, H, (C₂-C₆) Alkoxyalkyl, (C₂-C₆) Haloalkoxyalkyl, (C₁-C₆) Alkyl, (C₁-C₆) Haloalkyl, (C₁-C₆) Cyanoalkyl, (C₁-C₆) Alkylthio, (C₂-C₆) Alkylthioalkyl, (C₂-C₆) Alkylsulfinylalkyl, (C₂-C₆) Alkylsulfonylalkyl, (C₇-C₈) Arylalkyl wie Benzyl, (C₂-C₆) Alkenyl, (C₂-C₆) Haloalkenyl, (C₂-C₆) Alkinyl, (C₂-C₆) Haloalkinyl, Aryl wie Naphthyl oder Phenyl, das ein- oder mehrfach substituiert sein kann mit Halogen, (C₁-C₃) Alkyl oder (C₁-C₃) Alkoxy, Heteroaryl wie Pyridin, wobei
R¹ und R² auch zu einem Ring verbunden sein können, so daß sich Struktur (II) ergibt, 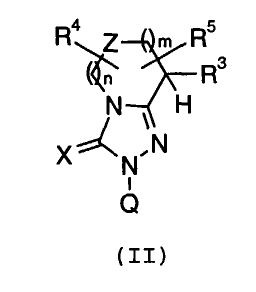 worin
- n und m: unabhängig voneinander 0, 1, 2 oder 3 sind,
- Z =: CR⁴R⁵, O, S, S(O), S(O)₂, N((C₁-C₄)Alkyl), N((C₁-C₄)Haloalkyl), C=O, C=N-R⁴ oder C=S,
- R⁴ =: H, (C₁-C₃) Alkyl, Halogen, (C₁-C₆) Alkoxy, (C₁-C₆) Haloalkyl, (C₁-C₆) Haloalkoxy, (C₂-C₆) Alkylcarbonyloxy oder (C₂-C₆) Haloalkylcarbonyloxy,
- R⁵ =: H, (C₁-C₃) Alkyl oder Halogen,
wobei R⁴ und R⁵, unabhängig voneinander, ein- oder mehrfach den Ring substituieren können und bis zu 12 (m = 3, n = 3) Positionen einnehmen können,
bedeuten,
Q für einen der Reste 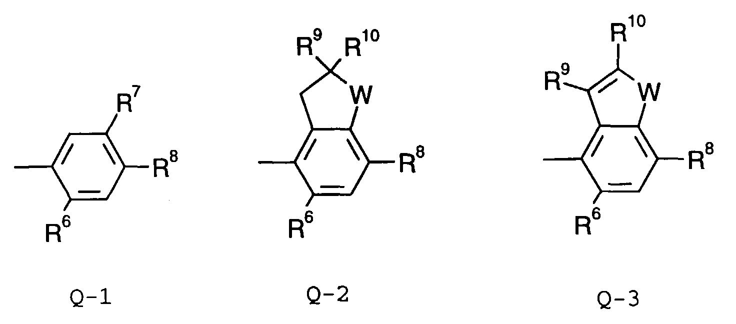 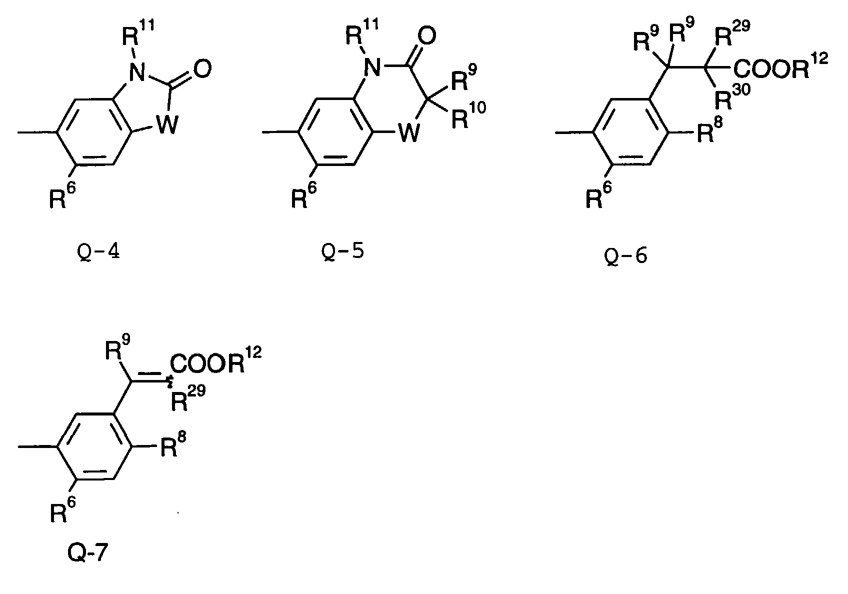 steht, wobei
- W =: O oder S,
- R⁶ =: H, Halogen,
- R⁷ =: H, (C₁-C₈) Alkyl, (C₁-C₈) Haloalkyl, Halogen, OH, OR¹², SH, S(O)ₚR¹², COR¹², CO₂R¹², C(O)SR¹², C(O)NR¹⁴R¹⁵, CHO, CR¹⁴=NOR²¹, CHCR²²CO₂R¹², CH₂CHR²²CO₂R¹², CO₂N=CR¹⁶R¹⁷, NO₂, CN, NHSO₂R¹⁸, NHSO₂NHR¹⁸, NR¹²R²³, NH₂ oder Phenyl, wahlweise substituiert mit R²⁴,
- p =: 0,1 oder 2,
- R⁸ =: (C₁-C₂) Alkyl, (C₁-C₂) Haloalkyl, OCH₃, SCH₃, OCHF₂, Halogen, CN oder NO₂,
- R⁹ =: H, (C₁-C₃) Alkyl oder Halogen,
- R¹⁰ =: H, (C₁-C₃) Alkyl, Halogen, (C₁-C₃) Haloalkyl, Cyclopropyl, Vinyl, C₂-Alkinyl, CN, C(O)R²³,CO₂R²³, C(O)NR²³R²⁵, CR¹⁹R²⁰CN, CR¹⁹R²⁰C(O)R²³, CR¹⁹R²⁰CO₂R²³, CR¹⁹R²⁰C(O)NR²³R²⁵, CHR¹⁹OH, CHR¹⁹OC(O)R²³ oder OCHR¹⁹OC(O)NR²³R²⁵, oder, wenn Q gleich Q-2 ist, können R⁹ und R¹⁰ zusammen mit dem Kohlenstoffatom, an dem sie gebunden sind, C=O sein,
- R¹¹ =: H, (C₁-C₆) Alkyl, (C₁-C₆) Haloalkyl, (C₃-C₆) Haloalkenyl, (C₂-C₆) Alkoxyalkyl, (C₃-C₆) Alkenyl, (C₃-C₆) Alkinyl, (C₃-C₆) Haloalkinyl, (C₄-C₇) Cycloalkylalkyl, 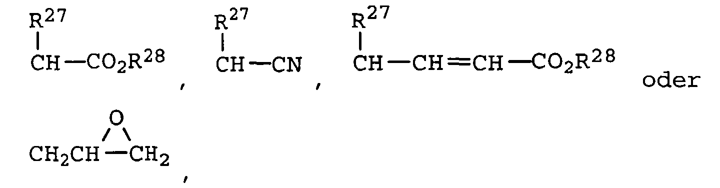
- R¹² =: (C₁-C₈) Alkyl, (C₃-C₈) Cycloalkyl, (C₃-C₈) Alkenyl, (C₃-C₈) Alkinyl, (C₁-C₈) Haloalkyl, (C₂-C₈) Alkoxyalkyl, (C₂-C₈) Alkylthioalkyl, (C₂-C₈) Alkylsulfinylalkyl, (C₂-C₈) Alkylsulfonylalkyl, (C₄-C₈) Alkoxyalkoxyalkyl, (C₄-C₈) Cycloalkylalkyl, (C₆-C₈) Cycloalkoxyalkyl, (C₄-C₈) Alkenyloxyalkyl, (C₄-C₈) Alkinyloxyalkyl, (C₃-C₈) Haloalkoxyalkyl, (C₄-C₈) Haloalkenyloxyalkyl, (C₄-C₈) Haloalkinyloxyalkyl, (C₆-C₈) Cycloalkylthioalkyl, (C₄-C₈) Alkenylthioalkyl, (C₄-C₈) Alkinylthioalkyl, (C₁-C₄) Alkyl, substituiert mit Phenoxy oder Benzyloxy, jeder Ring wahlweise substituiert mit Halogen, (C₁-C₃) Alkyl oder (C₁-C₃) Haloalkyl, (C₄-C₈) Trialkylsilylalkyl, (C₃-C₈) Cyanoalkyl, (C₃-C₈) Halocycloalkyl, (C₃-C₈) Haloalkenyl, (C₅-C₈) Alkoxyalkenyl, (C₅-C₈) Haloalkoxyalkenyl, (C₅-C₈) Alkylthioalkenyl, (C₃-C₈) Haloalkinyl, (C₅-C₈) Alkoxyalkinyl, (C₅-C₈) Haloalkoxyalkinyl, (C₅-C₈) Alkylthioalkinyl, (C₂-C₈) Alkylcarbonyl, Benzyl, wahlweise substituiert mit Halogen, (C₁-C₃) Alkyl oder (C₁-C₃) Haloalkyl, CHR¹⁹COR¹³, CHR¹⁹P(O)(OR¹³)₂, CHR¹⁹P(S)(OR¹³)₂, P(O)(OR¹³)₂, P(S)(OR¹³)₂, CHR¹⁹C(O)NR¹⁴R¹⁵, CHR¹⁹C(O)NH₂, CHR¹⁹CO₂R¹³, CO₂R¹³, SO₂R¹³, Phenyl, wahlweise substituiert mit R²⁴, 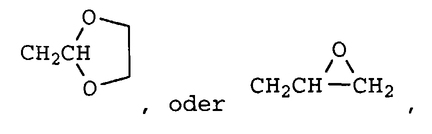
- R¹³ =: (C₁-C₆) Alkyl, (C₁-C₆) Haloalkyl, (C₃-C₆) Alkenyl oder (C₃-C₆) Alkinyl,
- R¹⁴ und R¹⁶ =: unabhängig voneinander, H oder (C₁-C₄) Alkyl,
- R¹⁵ und R¹⁷ =: unabhängig voneinander, (C₁-C₄) Alkyl oder Phenyl, wahlweise substituiert mit Halogen, (C₁-C₃) Alkyl oder (C₁-C₃) Haloalkyl, oder
- R¹⁴ und R¹⁵: können zusammen mit dem Stickstoffatom, das sie verbindet, einen Piperidinyl-, Pyrrolidinyl- oder Morpholinyl-Ring bilden, jeder Ring wahlweise substituiert mit (C₁-C₃) Alkyl, Phenyl oder Benzyl, oder
- R¹⁶ und R¹⁷: können zusammen mit dem Kohlenstoffatom, das sie verbindet, (C₃-C₈) Cycloalkyl sein,
- R¹⁸ =: (C₁-C₄) Alkyl oder (C₁-C₄) Haloalkyl,
- R¹⁹ und R²⁰ =: unabhängig H oder (C₁-C₅) Alkyl,
- R²¹ =: H, (C₁-C₆) Alkyl, (C₃-C₆) Alkenyl oder (C₃-C₆) Alkinyl,
- R²² und R²⁷ =: unabhängig voneinander, H, (C₁-C₄) Alkyl oder Halogen, oder
- R¹² und R²²: können zusammen ein (C₂-C₃) Alkylen bilden,
- R²³, R²⁴ und R²⁸ =: unabhängig voneinander, H oder (C₁-C₄) Alkyl,
- R²⁵ =: (C₁-C₂) Alkyl, (C₁-C₂) Haloalkyl, OCH₃, SCH₃, OCHF₂, Halogen, CN oder NO₂ und
- R²⁶ =: H, (C₁-C₅) Alkyl, (C₁-C₅) Haloalkyl, (C₃-C₆) Cycloalkyl, (C₃-C₆) Halocycloalkyl, (C₁-C₅) Alkoxy, (C₁-C₅) Haloalkoxy und Phenyl, das ggf. bis zu dreifach wahlweise mit Halogen, NO₂, Cyano, (C₁-C₂) Alkyl, (C₁-C₂) Haloalkyl, (C₁-C₂) Alkoxy oder (C₁-C₂) Haloalkoxy substituiert sein kann,
- R²⁹ =: H, Halogen oder (C₁-C₆) Alkyl,
- R³⁰ =: H, (C₁-C₄) Alkyl oder Halogen,
bedeutet.

Die Verbindungen der Formeln I oder II sind hochwirksame Herbizide, die als Protoporphyrinogen-Oxidase Inhibitor wirken und selbst bei geringen Aufwandmengen Ungräser und Unkräuter kontrollieren. Von großem Interesse sind I und II z. B. für Kulturen mit Plantagenanbau, wo schnell wachsende und robuste Schadpflanzen die Ernte beeinträchtigen. Durch die ausgezeichnete Selektivität des Typs I oder II können diese Verbindungen jedoch auch in Kulturpflanzen wie Mais, Soja, Weizen oder Gerste zur Bekämpfung von Schadpflanzen Anwendung finden.

Bei bekannten Verfahren zur Herstellung der Verbindung I geht man in der Regel von einem geeignet substituierten Phenylhydrazin aus, das durch Reaktion mit einem *N*-(1-Alkoxy)alkyliden-alkylcarbamat oder mit einem α-Ketocarbonsäurederivat und durch anschließende Schmidt-Umlagerung in die entsprechenden Triazolidinone überführt wird.

So beschreibt EP 0 220 952 die in Xylol durchgeführte Überführung von 2-Chlorophenylhydrazin mit *N*-(1-Ethoxy)ethyliden-ethylcarbamat in das in 4-Stellung unsubstituierte Triazolinon, das anschließend in einem eigenen Reaktionsschritt in Dimethylformamid zum trisubstituierten Triazolinon alkyliert wird. Bicyclische Triazolinone sind so nur sehr aufwendig herstellbar.

US-A-4 818 275 und US-A-4 818 276 beschreiben ebenfalls Verfahren zu substituierten Triazolinonen. Durch saure Kondensation des eingesetzten Phenylhydrazins mit einer α-Ketocarbonsäure entsteht zunächst das Hydrazon, welches durch Reaktion mit Diphenylphosphorylazid eine Schmidt-Umlagerung erfährt und so in das Triazolinon überführt wird. Das entstandene Triazolinon muß ebenfalls in einem weiteren Reaktionsschritt zur Endverbindung alkyliert werden. Bicyclische Triazolinone sind so ebenfalls nur sehr aufwendig synthetisierbar und das eingesetzte Azid birgt sicherheitstechnische Probleme bei einer industriellen Synthese der Verbindungen.

Bei weiteren bekannten Verfahren zur Herstellung einzelner Vertreter des Verbindungstyps II verläuft die Reaktion meist über das entsprechende Amidrazon (III), das zu bicyclischen Verbindungen umgesetzt wird.

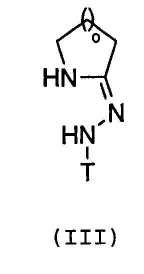

In DE 28 01 429 wird ein Verfahren zur Herstellung von Verbindungen des Typs II beschrieben, bei dem man 2-Piperidon, das zunächst nach literaturbekannten Methoden (vgl. Houben-Weyl, "Methoden der organischen Chemie", Bd. 11/2, S. 578; ferner DE-OS 19 12 739 und DE-OS 19 12 737) geeignet aktiviert werden muß, mit dem HCl-Salz des entsprechend substituierten Phenylhydrazins zum Amidrazon (III) (vgl. DT-OS 22 35 177) reagieren läßt und das entstandene Amidrazon mit Chlorameisensäuremethylester bzw. Phosgen in THF unter Zusatz von Base zu Verbindungen des Typs II cyclisiert.

In einem weiteren Verfahren (vgl. PCT/WO 94/22828) wird als aktivierte Zwischenverbindung das *N*-Carboxymethyl-2-iminopiperidin-Hydrochlorid beschrieben. Die hydrolyseempfindliche Zwischenverbindung wird mit dem entsprechend substituierten Phenylhydrazin gekoppelt und anschließend säurekatalytisch zum Triazolinon cyclisiert.

Das Verfahren gemäß EP 0 317 947 A2 zur Synthese von Triazolinonen beinhaltet die Umsetzung von *N*-Carboxyalkyl-Lactamen mit einem Phenylhydrazinen zum Amidrazon durch Kochen unter Rückfluß in Xylol. Durch Reaktion mit Phosgen entsteht das bicyclische Triazolinon. Das Verfahren hat den Nachteil, daß der Kondensationsschritt zum Amidrazon mit nur 21 % Ausbeute in Gegenwart von P₄O₁₀ als wasserentziehendes Mittel verläuft.

Die aufgeführten bekannten Verfahren haben die Nachteile, daß sie z. T. nur über lange Synthesewege, die z. T. über aufwendig herzustellende und zu behandelnde Zwischenstufen führen, realisierbar sind, weiterhin moderate Ausbeuten liefern sowie z. T. teure und nicht immer gefahrlose Einsatzstoffe verwenden und verschiedene Nebenprodukte entstehen, die aufwendige Reinigungsstufen zur Folge haben.

Aufgabe der Erfindung ist demnach, ein Verfahren zur Verfügung zu stellen, das die oben genannten Nachteile nicht besitzt und ökonomisch attraktiver ist.

Diese Aufgabe zur Herstellung von Verbindungen des Typs (I), 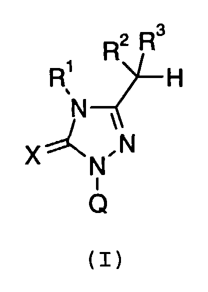 worin
- X =: O oder S,
- R¹ =: (C₂-C₈) Alkoxyalkyl, (C₂-C₈) Haloalkoxyalkyl, (C₁-C₈) Alkyl, (C₁-C₈) Haloalkyl, (C₁-C₈) Cyanoalkyl, (C₂-C₈) Alkylthioalkyl, (C₂-C₈) Alkylsulfinylalkyl, (C₂-C₈) Alkylsulfonylalkyl, (C₇-C₈) Arylalkyl wie Benzyl, (C₂-C₈) Alkenyl, (C₂-C₈) Haloalkenyl, (C₂-C₈) Alkinyl, (C₂-C₈) Haloalkinyl, Aryl wie Naphthyl oder Phenyl, das ein- oder mehrfach mit Halogen, (C₁-C₃) Alkyl oder (C₁-C₃) Alkoxy substituiert sein kann, Heteroaryl wie Pyridin, wobei Heteroaryl ggf. mit Halogen substituiert sein kann, bedeuten,
- R² und R³,: unabhängig voneinander, H, (C₂-C₆) Alkoxyalkyl, (C₂-C₆) Haloalkoxyalkyl, (C₁-C₆) Alkyl, (C₁-C₆) Haloalkyl, (C₁-C₆) Cyanoalkyl, (C₁-C₆) Alkylthio, (C₂-C₆) Alkylthioalkyl, (C₂-C₆) Alkylsulfinylalkyl, (C₂-C₆) Alkylsulfonylalkyl, (C₇-C₈) Arylalkyl wie Benzyl, (C₂-C₆) Alkenyl, (C₂-C₆) Haloalkenyl, (C₂-C₆) Alkinyl, (C₂-C₆) Haloalkinyl, Aryl wie Naphthyl oder Phenyl, das ein- oder mehrfach substituiert sein kann mit Halogen, (C₁-C₃) Alkyl oder (C₁-C₃) Alkoxy, Heteroaryl wie Pyridin, wobei
R¹ und R² auch zu einem Ring verbunden sein können, so daß sich Struktur (II) ergibt, 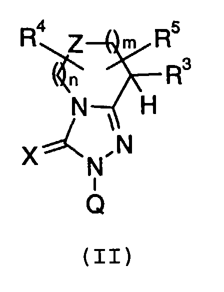 worin
- n und m: unabhängig voneinander 0, 1, 2 oder 3 sind,
- Z =: CR⁴R⁵, O, S, S(O), S(O)₂, N((C₁-C₄)Alkyl), N((C₁-C₄)Haloalkyl), C=O, C=N-R⁴ oder C=S,
- R⁴ =: H, (C₁-C₃) Alkyl, Halogen, (C₁-C₆) Alkoxy, (C₁-C₆) Haloalkyl, (C₁-C₆) Haloalkoxy, (C₂-C₆) Alkylcarbonyloxy oder (C₂-C₆) Haloalkylcarbonyloxy,
- R⁵ =: H, (C₁-C₃) Alkyl oder Halogen,
wobei R⁴ und R⁵, unabhängig voneinander, ein- oder mehrfach den Ring substituieren können und bis zu 12 (m = 3, n = 3) Positionen einnehmen können,
bedeuten,
Q für einen der Reste 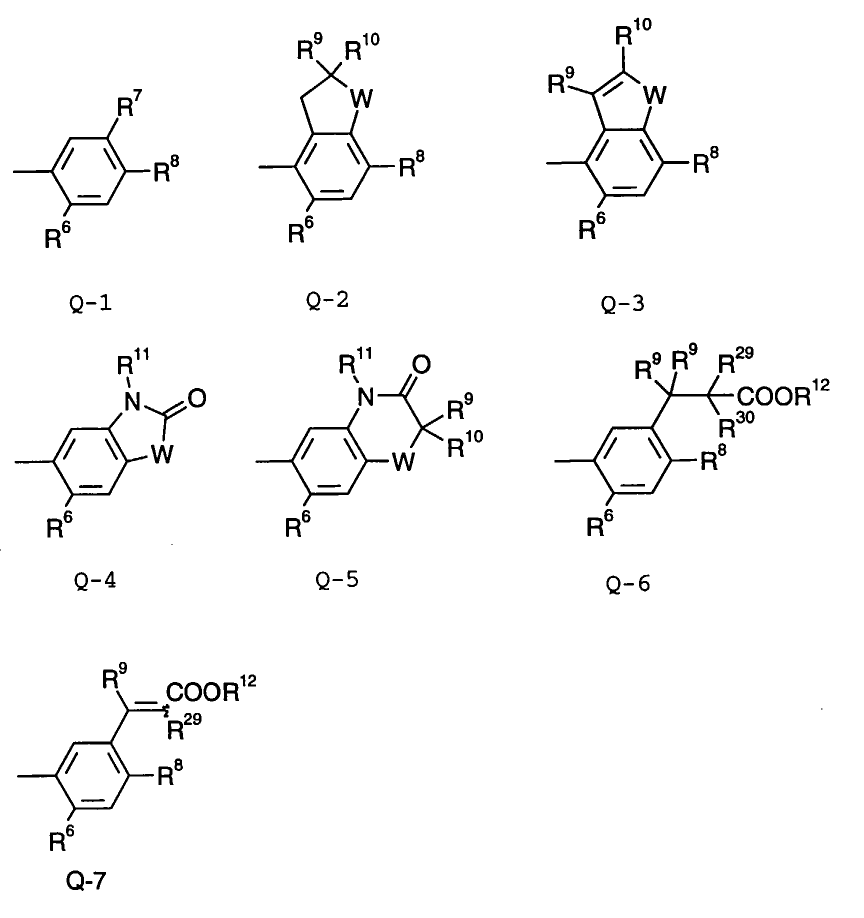 steht, wobei
- W =: O oder S,
- R⁶ =: H, Halogen,
- R⁷ =: H, (C₁-C₈) Alkyl, (C₁-C₈) Haloalkyl, Halogen, OH, OR¹², SH, S(O)ₚR¹², COR¹², CO₂R¹², C(O)SR¹², C(O)NR¹⁴R¹⁵, CHO, CR¹⁴=NOR²¹, CHCR²²CO₂R¹², CH₂CHR²²CO₂R¹², CO₂N=CR¹⁶R¹⁷, NO₂, CN, NHSO₂R¹⁸, NHSO₂NHR¹⁸, NR¹²R²³, NH₂ oder Phenyl, wahlweise substituiert mit R²⁴,
- p =: 0,1 oder 2,
- R⁸ =: (C₁-C₂) Alkyl, (C₁-C₂) Haloalkyl, OCH₃, SCH₃, OCHF₂, Halogen, CN oder NO₂,
- R⁹ =: H, (C₁-C₃) Alkyl oder Halogen,
- R¹⁰ =: H, (C₁-C₃) Alkyl, Halogen, (C₁-C₃) Haloalkyl, Cyclopropyl, Vinyl, C₂-Alkinyl, CN, C(O)R²³,CO₂R²³, C(O)NR²³R²⁵, CR¹⁹R²⁰CN, CR¹⁹R²⁰C(O)R²³, CR¹⁹R²⁰CO₂R²³, CR¹⁹R²⁰C(O)NR²³R²⁵, CHR¹⁹OH, CHR¹⁹OC(O)R²³ oder OCHR¹⁹OC(O)NR²³R²⁵, oder, wenn Q gleich Q-2 ist, können R⁹ und R¹⁰ zusammen mit dem Kohlenstoffatom, an dem sie gebunden sind, C=O sein,
- R¹¹ =: H, (C₁-C₆) Alkyl, (C₁-C₆) Haloalkyl, (C₃-C₆) Haloalkenyl, (C₂-C₆) Alkoxyalkyl, (C₃-C₆) Alkenyl, (C₃-C₆) Alkinyl, (C₃-C₆) Haloalkinyl, (C₄-C₇) Cycloalkylalkyl, 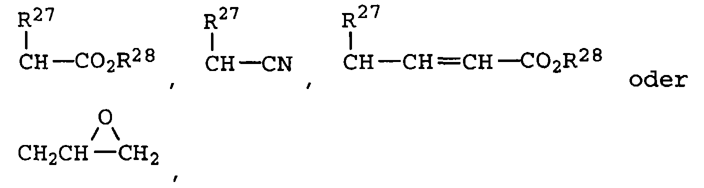
- R¹² =: (C₁-C₈) Alkyl, (C₃-C₈) Cycloalkyl, (C₃-C₈) Alkenyl, (C₃-C₈) Alkinyl, (C₁-C₈) Haloalkyl, (C₂-C₈) Alkoxyalkyl, (C₂-C₈) Alkylthioalkyl, (C₂-C₈) Alkylsulfinylalkyl, (C₂-C₈) Alkylsulfonylalkyl, (C₄-C₈) Alkoxyalkoxyalkyl, (C₄-C₈) Cycloalkylalkyl, (C₆-C₈) Cycloalkoxyalkyl, (C₄-C₈) Alkenyloxyalkyl, (C₄-C₈) Alkinyloxyalkyl, (C₃-C₈) Haloalkoxyalkyl, (C₄-C₈) Haloalkenyloxyalkyl, (C₄-C₈) Haloalkinyloxyalkyl, (C₆-C₈) Cycloalkylthioalkyl, (C₄-C₈) Alkenylthioalkyl, (C₄-C₈) Alkinylthioalkyl, (C₁-C₄) Alkyl, substituiert mit Phenoxy oder Benzyloxy, jeder Ring wahlweise substituiert mit Halogen, (C₁-C₃) Alkyl oder (C₁-C₃) Haloalkyl, (C₄-C₈) Trialkylsilylalkyl, (C₃-C₈) Cyanoalkyl, (C₃-C₈) Halocycloalkyl, (C₃-C₈) Haloalkenyl, (C₅-C₈) Alkoxyalkenyl, (C₅-C₈) Haloalkoxyalkenyl, (C₅-C₈) Alkylthioalkenyl, (C₃-C₈) Haloalkinyl, (C₅-C₈) Alkoxyalkinyl, (C₅-C₈) Haloalkoxyalkinyl, (C₅-C₈) Alkylthioalkinyl, (C₂-C₈) Alkylcarbonyl, Benzyl, wahlweise substituiert mit Halogen, (C₁-C₃) Alkyl oder (C₁-C₃) Haloalkyl, CHR¹⁹COR¹³, CHR¹⁹P(O)(OR¹³)₂, CHR¹⁹P(S)(OR¹³)₂, P(O)(OR¹³)₂, P(S)(OR¹³)₂, CHR¹⁹C(O)NR¹⁴R¹⁵, CHR¹⁹C(O)NH₂, CHR¹⁹CO₂R¹³, CO₂R¹³, SO₂R¹³, Phenyl, wahlweise substituiert mit R²⁴, 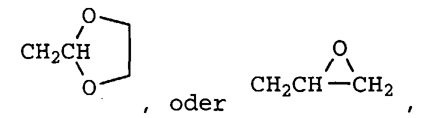
- R¹³ =: (C₁-C₆) Alkyl, (C₁-C₆) Haloalkyl, (C₃-C₆) Alkenyl oder (C₃-C₆) Alkinyl,
- R¹⁴ und R¹⁶ =: unabhängig voneinander, H oder (C₁-C₄) Alkyl,
- R¹⁵ und R¹⁷ =: unabhängig voneinander, (C₁-C₄) Alkyl oder Phenyl, wahlweise substituiert mit Halogen, (C₁-C₃) Alkyl oder (C₁-C₃) Haloalkyl, oder
- R¹⁴ und R¹⁵: können zusammen mit dem Stickstoffatom, das sie verbindet, einen Piperidinyl-, Pyrrolidinyl- oder Morpholinyl-Ring bilden, jeder Ring wahlweise substituiert mit (C₁-C₃) Alkyl, Phenyl oder Benzyl, oder
- R¹⁶ und R¹⁷: können zusammen mit dem Kohlenstoffatom, das sie verbindet, (C₃-C₈) Cycloalkyl sein,
- R¹⁸ =: (C₁-C₄) Alkyl oder (C₁-C₄) Haloalkyl,
- R¹⁹ und R²⁰ =: unabhängig H oder (C₁-C₅) Alkyl,
- R²¹ =: H, (C₁-C₆) Alkyl, (C₃-C₆) Alkenyl oder (C₃-C₆) Alkinyl,
- R²² und R²⁷ =: unabhängig voneinander, H, (C₁-C₄) Alkyl oder Halogen, oder
- R¹² und R²²: können zusammen ein (C₂-C₃) Alkylen bilden,
- R²³, R²⁴ und R²⁸ =: unabhängig voneinander, H oder (C₁-C₄) Alkyl,
- R²⁵ =: (C₁-C₂) Alkyl, (C₁-C₂) Haloalkyl, OCH₃, SCH₃, OCHF₂, Halogen, CN oder NO₂ und
- R²⁶ =: H, (C₁-C₅) Alkyl, (C₁-C₅) Haloalkyl, (C₃-C₆) Cycloalkyl, (C₃-C₆) Halocycloalkyl, (C₁-C₅) Alkoxy, (C₁-C₅) Haloalkoxy und Phenyl, das ggf. bis zu dreifach wahlweise mit Halogen, NO₂, Cyano, (C₁-C₂) Alkyl, (C₁-C₂) Haloalkyl, (C₁-C₂) Alkoxy oder (C₁-C₂) Haloalkoxy substituiert sein kann,
- R²⁹ =: H, Halogen oder (C₁-C₆) Alkyl,
- R³⁰ =: H, (C₁-C₄) Alkyl oder Halogen,
bedeutet,
wird beim erfindungsgemäßen Verfahren dadurch gelöst, daß Amide der allgemeinen Formel (IV) 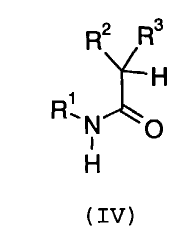 worin R¹, R² und R³ die oben angegebene Bedeutung besitzen, in an sich bekannter Weise mit Phosgen bzw. Thiophosgen oder einem Phosgenersatz bzw. Thiophosgenersatz wahlweise in einem Lösungsmittel zu einer Verbindung der Formel (V) 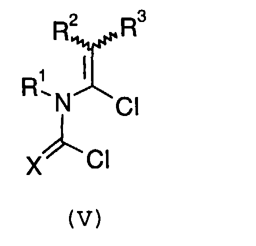 umgesetzt werden, worin R¹, R², R³ und X die oben angegebene Bedeutung besitzen, und diese Verbindungen der Formel (V) wird anschließend mit einer Verbindung der Formel (VI) oder ein Säureadditionssalz hiervon,

Q―NH-NH-CO-R²⁶ (VI)

worin Q und R²⁶ die oben angegebene Bedeutung besitzen, wahlweise in Gegenwart eines Verdünnungsmittels und wahlweise in Gegenwart einer Base und anschließend in Gegenwart einer Säure und wahlweise durch Temperaturerhöhung zu einer Verbindung der Formeln (I) oder (II) umsetzt.

Bevorzugt können gemäß dem erfindungsgemäßen Verfahren Verbindungen des Typs II 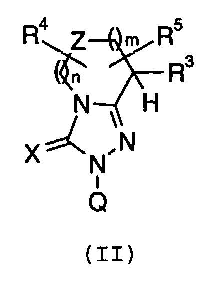 hergestellt werden, wobei
- X =: 0,
- n und m: unabhängig voneinander 0, 1 oder 2 sind,
- Z =: CR⁴R⁵, O, S, C=O oder C=S,
- R⁴ =: H, (C₁-C₂) Alkyl, (C₁-C₂) Haloalkyl, Fluor, Chlor, (C₁-C₃) Alkoxy oder (C₁-C₃) Haloalkoxy,
- R³ und R⁵: können unabhängig voneinander H, (C₁-C₂) Alkyl, Fluor oder Chlor sein,
- Q =: Q-1, Q-2 oder Q-5,
- R⁷ =: H, (C₁-C₈) Alkyl, (C₁-C₈) Haloalkyl, Halogen, OH, OR¹², SH, S(O)ₚR12, COR¹², CO₂R¹², C(O)SR¹², C(O)NR¹⁴R¹⁵, CHO, CH=CHCO₂R¹², CO₂N=CR¹⁶R¹⁷, NO₂, CN, NHSO₂R¹⁸ oder NHSO₂NHR¹⁵ und
- R¹² =: (C₁-C₄) Alkyl, (C₃-C₄) Alkenyl, (C₃-C₄) Alkinyl, (C₂-C₄) Alkoxyalkyl, (C₁-C₄) Haloalkyl, (C₃-C₄) Haloalkenyl oder (C₃-C₄) Haloalkinyl,
- R²⁶ =: H
bedeutet,
indem man Amide der allgemeinen Formel VII 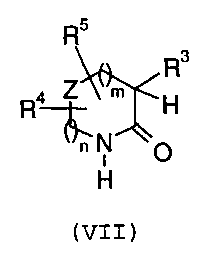 worin n, m, Z, R³, R⁴ und R⁵ die oben angegebene Bedeutung haben, in an sich bekannter Weise mit Phosgen bzw. Thiophosgen oder einem Phosgenersatz bzw. Thiophosgenersatz wahlweise in einem Lösungsmittel zu einer Verbindung der Formel VIII 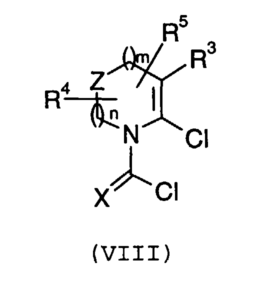 umsetzt, worin n, m, X, Z, R³, R⁴ und R⁵ die oben angegebene Bedeutung besitzen und diese Verbindung der Formel (VIII) anschließend mit einer Verbindung der Formel (VI) oder eines Säureadditionssalzes hiervon

Q―NH-NH-CO-R²⁶ (VI)

worin Q und R²⁶ die oben angegebene Bedeutung besitzen, wahlweise in Gegenwart eines Verdünnungsmittels und wahlweise zunächst in Gegenwart einer Base und anschließend in Gegenwart einer Säure und wahlweise durch Temperaturerhöhung zu einer Verbindung der Formel (II) umsetzt.

Ganz besonders bevorzugt ist ein Verfahren zur Herstellung von Verbindungen des Typs II, 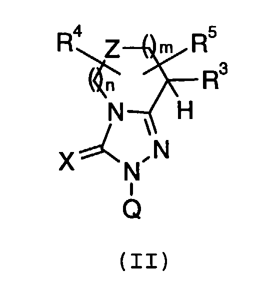 worin
- X =: 0,
- n und m: unabhängig voneinander 0, 1 oder 2 sind,
- Z =: CH₂, CHF, CF₂ oder CHCl,
- R⁴ =: H, (C₁-C₂) Alkyl, (C₁-C₂) Haloalkyl, Fluor, Chlor ist,
- R⁵ =: H, (C₁-C₂) Alkyl, Fluor, Chlor ist,
- R³ =: H ist,
- Q =: Q-1 oder Q-5 ist,
- R⁷ =: H, (C₁-C₈) Alkyl, (C₁-C₈) Haloalkyl, Halogen, OH, OR¹², SH, S(O)ₚR¹², COR¹², CO₂R¹², C(O)SR¹², C(O)NR¹⁴R¹⁵, CHO, CH=CHCO₂R¹², CO₂N=CR¹⁶R¹⁷, NO₂, CN, NHSO₂R¹⁸ oder NHSO₂NHR¹⁵ und
- R¹² =: (C₁-C₄) Alkyl, (C₃-C₄) Alkenyl, (C₃-C₄) Alkinyl, (C₂-C₄) Alkoxyalkyl, (C₁-C₄) Haloalkyl, (C₃-C₄) Haloalkenyl oder (C₃-C₄) Haloalkinyl bedeuten, und
- R⁸ =: Halogen, CN oder NO₂
- R²⁶ =: H
bedeuten,
indem man Amide der allgemeinen Formel (VII) 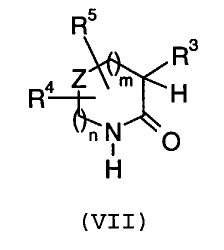 worin n, m, X, Z, R³, R⁴ und R⁵ die oben angegebene Bedeutung haben, in an sich bekannter Weise mit Phosgen oder Phosgenersatz zu Verbindungen des Typs (VIII) 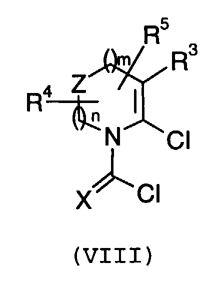 umgesetzt werden und diese Verbindung des Typs (VIII) anschließend mit einer Verbindung der Formel (VI) oder eines Säureadditionssalzes hiervon

Q―NH-NH-CO-R²⁶ (VI)

worin Q und R²⁶ die oben angegebene Bedeutung besitzen, wahlweise in Gegenwart eines Verdünnungsmittels und wahlweise in Gegenwart einer Base und anschließend in Gegenwart einer Säure und wahlweise durch Temperaturerhöhung zu einer Verbindung der Formel (II) umsetzt.

Ganz besonders bevorzugt ist das erfindungsgemäße Verfahren zur Herstellung der Verbindung (IX) 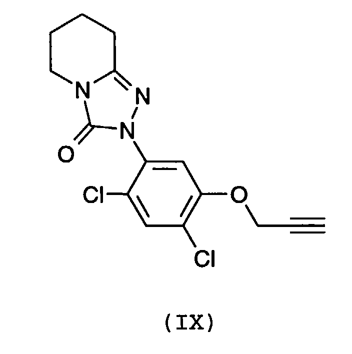 indem man 2-Piperidon mit Phosgen oder einem Phosgenersatz wahlweise in einem Lösungsmittel in an sich bekannter Weise zu einer Verbindung der Formel (X) 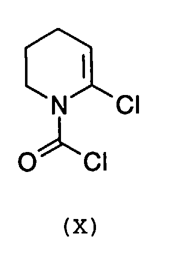 umsetzt, und diese Verbindung der Formel (X)
anschließend mit einer Verbindung der Formel (XI)
oder eines Säureadditionssalzes hiervon, 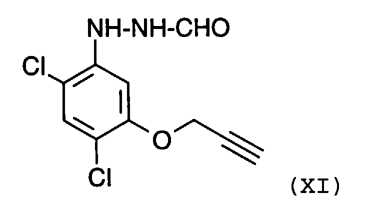 wahlweise in Gegenwart eines Verdünnungsmittels und wahlweise in Gegenwart einer Base und anschließend in Gegenwart einer Säure und wahlweise durch Temperaturerhöhung zu einer Verbindung der Formel (IX) reagieren läßt.

Unter der Bezeichnung "Alkylgruppen" sind sowohl "geradkettige" als auch "verzweigte" Alkylgruppen zu verstehen. Unter der Bezeichnung "geradkettige Alkylgruppe" sind beispielsweise Reste wie Methyl, Ethyl, n-Propyl, n-Butyl, n-Pentyl, n-Hexyl, unter "verzweigter Alkylgruppe" Reste wie beispielsweise Isopropyl oder tert.-Butyl zu verstehen. Unter "Cycloalkyl" sind Reste wie beispielsweise Cyclopropyl, Cyclobutyl, Cyclopentyl, Cyclohexyl oder Cycloheptyl zu verstehen. Die Bezeichnung Halogen steht für Fluor, Chlor, Brom oder Jod. Die Bezeichnung "Alkoxygruppe" stellt Reste wie beispielsweise Methoxy, Ethoxy, Propoxy, Butoxy, Isopropoxy, Isobutoxy oder Pentoxy dar.

Vorteilhafte Varianten des erfindungsgemäßen Verfahrens werden in den auf Anspruch 1 - 4 rückbezogenen Ansprüchen unter Schutz gestellt.

Eine bevorzugte Ausgestaltungsform ist eine Synthesevariante, bei der die Verbindung der allgemeinen Formel (V) 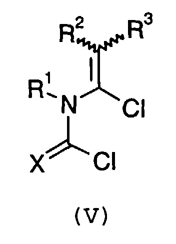 durch Umsetzung des Amids (IV) 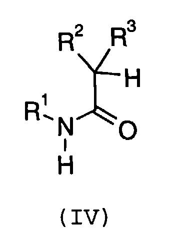 mit 2 - 6 Moläquivalenten Phosgen oder Thiophosgen bzw. Phosgenersatz oder Thiophosgenersatz, in einem inerten organischen Lösungsmittel, beispielsweise in einem aromatischen Lösungsmittel wie Toluol, Chlorbenzol, einem halogenierten Kohlenwasserstoff wie Chloroform, Methylenchlorid, einem Ether wie Diisopropylether, tert. Butylmethylether, Tetrahydrofuran oder Diethylether, Acetonitril oder Carbonsäurester, beispielsweise Essigsäureethylester, Essigsäuremethylester oder Essigsäureisopropylester, bei Temperaturen zwischen -20 °C bis +120 °C, vorzugsweise -20 °C bis +70 °C in literaturbekannterweise gemäß US-A-3,080,358 in hoher Ausbeute erhalten wird. Überschüssiges Phosgen bzw. Thiophosgen wird ausgetrieben und gegebenfalls das Lösungsmittel abgezogen. Bei 0-5°C gibt man die Verbindung der Formel (V), gegebenenfalls in einem Lösungsmittel gelöst zu einer Lösung der Verbindung der Formel (VI) oder eines Säureadditionssalzes von (VI), wie beispielsweise ein HCl-Salz oder ein H₂SO₄-Salz, im gleichen Lösungsmittel oder in einem anderen, oben genannten Lösungsmittel, optional in Gegenwart eines Säureakzeptors, beispielsweise einer organischen Base wie Triethylamin, Tetramethylpiperidin, Tributylamin oder Pyridin oder einer anorganischen Base wie NaOH, KOH, Ca(OH)₂, CaO, Na₂CO₃, K₂CO₃, CaCO₃, NaHCO₃, KHCO₃ oder Alkoholate wie NaOCH₃ oder NaOC₂H₅, zu, wobei zunächst die Verbindung (XV) entsteht, 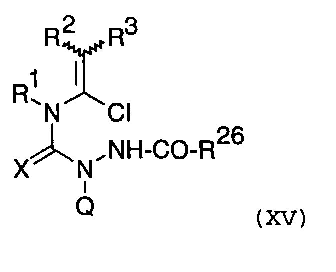 die dann optional in Gegenwart einer organischen oder anorganischen Säure wie beispielsweise HCl, H₂SO₄ HNO₃, HCO₂H, p-Toluolsulfonsäure, Essigsäure, Camphersulfonsäure oder H₃PO₄ oder eines sauren Ionenaustauschers und/oder unter Temperaturerhöhung, beispielsweise bis zum Siedepunkt des verwendeten Lösungsmittels, in die Verbindungen der Formel (I) bzw. (II) überführt werden kann. Vorzugsweise wird die Zyklisierung von (XV)zu (I) bzw. (II) unter Säurekatalyse und/oder Temperaturerhöhung durchgeführt. Folgereaktionen können optional ohne Zwischenisolierung im gleichen Lösungsmittel erfolgen.

Bei der Umsetzung zu der Verbindung (XV) werden vorzugsweise 1 - 4 Moläquivalente Base, besonders bevorzugt 1,5 - 3,5 Moläquivalente Base zugesetzt, wobei 1 Moläquivalent Base dazu dient, aus dem ggf. verwendeten Acylphenylhydrazin·Säureadditionssalz das korrespondierende Acylphenylhydrazin freizusetzen. Zur Überführung (Zyklisierung) der Verbindung (XV) in (I) ist ein gewisser Säureüberschuß notwendig. Es zeigt sich, daß die Reaktion umso stärker beschleunigt wird, je größer der Säureüberschuß ist. Die Menge an zugesetzter Säure orientiert sich an der zuvor verwendeten Menge an Base. Besonders bevorzugt als Säure sind H₂SO₄ und HCl. Eine Temperaturerhöhung, vorzugsweise bis zum Siedepunkt des Lösungsmittels, beschleunigt die Zyklisierung zu (I).

Im erfindungsgemäßen Verfahren wird durch eine geschickte Reaktionsführung die Amideinheit (IV) über die ökonomisch sehr interessante Phosgenierung bzw. Thiophosgenierung in einem Schritt doppelt aktiviert, so daß eine verfahrenstechnisch und ökonomisch wesentlich aufwendigere, schrittweise Aktivierung wie beispielsweise nach EP 0 220 952 vermieden werden kann.

Ebenfalls erfolgt im erfindungsmäßigen Verfahren die Reaktion zu (I) ausgehend von (V) und (VI) in einem Verfahrensschritt, ohne zwischenzeitig notwendige Aufarbeitung, in der gewünschten Art und Weise. Das bedeutet, daß gegenüber den bekannten Verfahren, wie beispielsweise in DE 28 01 429 beschrieben, ein deutlicher, verfahrenstechnischer Vorteil daraus erwächst, daß Kopplung, Abspaltung der Schutzgruppe und Zyklisierung zur Verbindung der Formel (I) in nur einem Schritt realisierbar ist.

Durch die beschriebene Vorgehensweise kann die Reaktion im Eintopfverfahren und ohne Isolierung von Zwischenstufen erfolgen, so daß aufwendige Aufarbeitungstechniken vermieden werden und bei dem erfindungsgemäßen Verfahren ein glatter Reaktionsverlauf ermöglicht wird und eine Nebenproduktbildung weitestgehend vermieden und die Gesamtausbeute an (I) erhöht wird. Ebenso kann die Reaktion unter Isolierung der Zwischenstufe (XV) erfolgen.

Amide der allgemeinen Formeln (IV) und (VII) sind kommerziell erhältlich oder darstellbar nach Houben-Weyl, "Methoden der Organischen Chemie", E5, "Carbonsäuren und Carbonsäure-Derivate", Teil 2, S. 934 ff.

Die Herstellung der Verbindung (XI) gelingt durch Formylierung der Verbindungen (XII) oder (XIII) 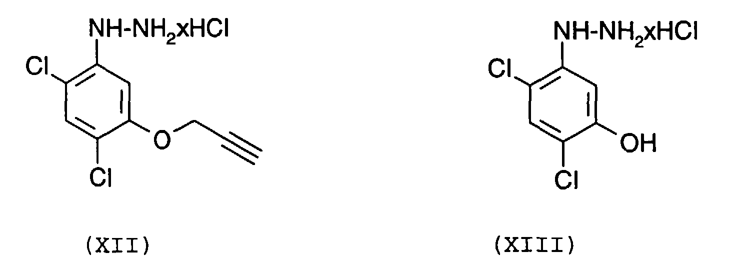 analog gängigen Literaturverfahren (siehe Houben-Weyl, 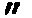 Methoden der Organ. Chemie", Bd 15I, S. 165ff; J. March 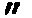 Advanced Organic Chemistry", 3. Auflage, S. 375ff), wobei aus (XIII) zunächst (XIV) 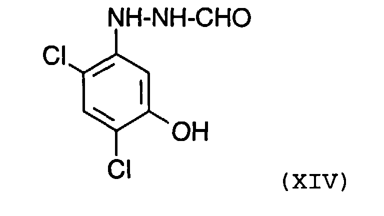 entsteht, das anschließend gemäß bekannten Literaturverfahren (Houben-Weyl, 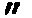 Methoden der Organ. Chemie", Bd. 6/3 I, S. 49ff und 54ff) zu (XI) noch verethert wird. Die Verbindung (XIII) kann ihrerseits durch bekannte Veretherungsreaktionen (Literaturstelle siehe oben) in (XII) überführt werden, bevor wie mit (XII) beschrieben, weiterverfahren wird.
Die Verbindungen (XI) und (XIV) sind wertvolle Zwischenverbindungen zur Synthese von Triazolinonen und in der Literatur bisher nicht beschrieben und damit neu.

Bevorzugt eignet sich das hier beanspruchte Verfahren zur Synthese bicyclischer Triazolinone der Formel (II) mit den oben als bevorzugte Strukturen angegebenen Substitutionen, weil diese Strukturen entweder besonders einfach und eindeutigt oder mit besonders hohen Ausbeuten entsprechend dem Verfahren zu Triazolinone reagieren. Die Reaktionsführung zur Herstellung von Verbindungen der allgemeinen Struktur (II) ist analog zu der bevorzugten Ausgestaltungsform zur Herstellung der Verbindungen der allgemeinen Formel (I). Die Zwischenisolierung der entsprechenden Zwischenverbindungen ist nicht nötig und ohne weitere Aufarbeitung läßt sich das Verfahren, ausgehend von (VI) und (XI), bis zur entsprechenden bevorzugten Verbindung der Formel (II) in einem Reaktionsgefäß ohne Wechsel des Lösungsmittels durchführen, wobei sich besonders Toluol, Acetonitril, Essigsäureethylester, Essigsäuremethylester, Essigsäureisopropylester Methylenchlorid und Chloroform für diese Umsetzung eignen. Die Umsetzung findet bevorzugt bei Temperaturen zwischen -20 °C und +70°C statt. Als bevorzugte Basen werden organische Basen wie Triethylamin, Tributylamin, Pyridin oder anorganische Basen wie Na₂CO₃, K₂CO₃, CaCO₃ eingesetzt. Bevorzugt eingesetzte Säuren sind HCl, H₂SO₄, H₃PO₄, Essigsäure, *p*-Toluolsulfonsäure.

Besonders bevorzugt eignet sich das beanspruchte Verfahren zur Synthese von 2-[2,4-Dichlor-5-(2-propynyloxy)phenyl]-5,6,7,8-tetrahydro-1,2,4-triazolo[4,3-a]pyridin-3(2H)-on der Formel (IX) 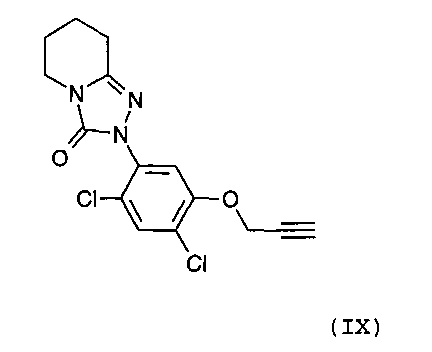 bei der die Verbindung der Formel (X) durch

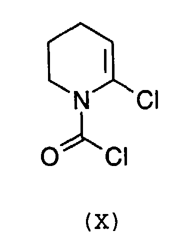

Umsetzung von 2-Piperidon mit 2 - 6 Moläquivalenten Phosgen oder Phosgenersatz, in einem inerten organischen Lösungsmittel, beispielsweise in einem aromatischen Lösungsmittel wie Toluol, Chlorbenzol, einem halogenierten Kohlenwasserstoff wie Chloroform, Methylenchlorid, einem Ether wie Diisopropylether, tert. Butylmethylether, Tetrahydrofuran oder Diethylether, Acetonitril oder Carbonsäurester, beispielsweise Essigsäureethylester, Essigsäuremethylester oder Essigsäureisopropylester, bei Temperaturen zwischen -20 °C bis +120 °C, vorzugsweise -20 °C bis +70 °C in literaturbekannterweise gemäß US-A-3,080,358 in über 94% iger Ausbeute erhalten wird. Überschüssiges Phosgen wird ausgetrieben und gegebenfalls das Lösungsmittel abgezogen. Bei 0-5°C gibt man die Verbindung der Formel (X), gegebenenfalls im gleichen Lösungsmittel oder einem anderen, oben genannten Lösungsmittel zu einer Lösung der Verbindung der Formel (XI) oder eines Säureadditionssalzes von (XI), wie beispielsweise ein HCl-Salz oder ein H₂SO₄-Salz 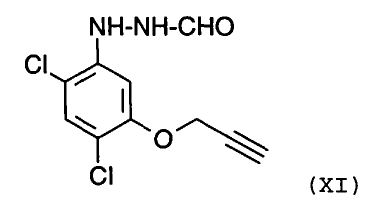 im gleichen Lösungsmittel oder wahlweise in einem anderen, oben genanten Lösungsmittel, optional in Gegenwart eines Säureakzeptors, beispielsweise einer organischen Base wie Triethylamin, Tetramethylpiperidin, Tributylamin oder Pyridin oder einer anorganischen Base wie NaOH, KOH, Ca(OH)₂, CaO, Na₂CO₃, K₂CO₃, CaCO₃, NaHCO₃, KHCO₃ oder Alkoholate wie NaOCH₃ oder NaOC₂H₅, zu, wobei zunächst die Verbindung (XVI) entsteht, 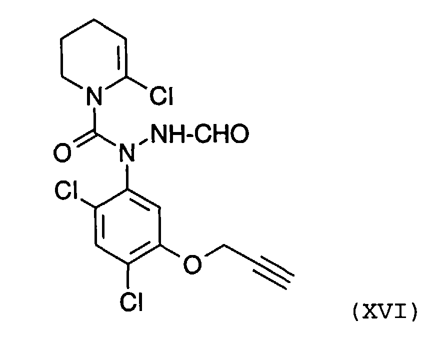 die dann optional in Gegenwart einer organischen oder anorganischen Säure wie beispielsweise HCl, H₂SO₄ HNO_{3,} HCO₂H, p-Toluolsulfonsäure, Essigsäure, Camphersulfonsäure oder H₃PO₄ oder eines sauren Ionenaustauschers und/oder unter Temperaturerhöhung, beispielsweise bis zum Siedepunkt des verwendeten Lösungsmittels, in die Verbindung der
Formel (IX) 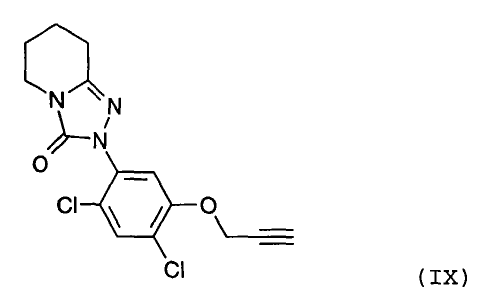 überführt wird. Vorzugsweise wird die Zyklisierung von (XVI) zu (IX) unter Säurekatalyse und/oder Temperaturerhöhung durchgeführt.

Bei der Umsetzung zu der Verbindung (XVI) werden vorzugsweise 1 - 4 Moläquivalente Base, besonders bevorzugt 1,5 - 3,5 Moläquivalente Base zugesetzt, wobei 1 Moläquivalent Base dazu dient, aus dem ggf. verwendeten Acylphenylhydrazin·Säureadditionssalz das korrespondierende Acylphenylhydrazin freizusetzen. Zur Überführung (Zyklisierung) der Verbindung (XVI) in (IX) ist ein gewisser Säureüberschuß notwendig. Es zeigt sich, daß die Reaktion umso stärker beschleunigt wird, je größer der Säureüberschuß ist. Die Menge an zugesetzter Säure orientiert sich an der zuvor verwendeten Menge an Base. Besonders bevorzugt als Säure sind H₂SO₄ und HCl. Eine Temperaturerhöhung, vorzugsweise bis zum Siedepunkt des Lösungsmittels, beschleunigt die Zyklisierung zu (IX).

Im erfindungsgemäßen Verfahren wird durch eine geschickte Reaktionsführung 2 - Piperidon über die ökonomisch sehr interesante Phosgenierung bzw. Thiophosgenierung in einem Schritt doppelt aktiviert, so daß eine verfahrenstechnisch und ökonomisch wesentlich aufwendigere, schrittweise Aktivierung wie beispielsweise nach EP 0 220 952 vermieden werden kann.

Ebenfalls erfolgt im erfindungsmäßigen Verfahren die Reaktion zu (IX) ausgehend von (X) und (XI) in einem Verfahrensschritt, ohne zwischenzeitig notwendige Aufarbeitung, in der gewünschten Art und Weise. Das bedeutet, daß gegenüber den bekannten Verfahren, wie beispielsweise in DE 28 01 429 beschrieben, ein deutlicher, verfahrenstechnischer Vorteil daraus erwächst, daß Kopplung, Abspaltung der Schutzgruppe und Zyklisierung zur Verbindung der Formel (IX) in nur einem Schritt realisierbar ist.

Durch die beschriebene Vorgehensweise kann die Reaktion ausgehend von den Verbindungen (X) und (XI) im Eintopfverfahren und ohne Isolation von Zwischenstufen erfolgen, so daß aufwendige Aufarbeitungstechniken vermieden werden und bei dem erfindungsgemäßen Verfahren ein glatter Reaktionsverlauf ermöglicht wird und eine Nebenproduktbildung weitestgehend vermieden und die Gesamtausbeute an (IX) erhöht wird. Ebenso kann die Reaktion unter Isolierung der Zwischenstufe (XVI) erfolgen.

Die Erfindung wird im weiteren anhand der Ausführungsbeispiele erläutert, ist aber nicht auf diese beschränkt.

### Beispiele

### Darstellung von 2-[2,4-Dichlor-5-(2-propinyloxy)phenyl]-5,6,7,8-tetrahydro-1,2,4-triazolo[4,3-a]pyridin-3(2H)-on und Vorstufensynthesen

### 1) N-Chlorcarbonyl-2-chlor-1,4,5,6-tetrahydropyridin

Diphosgen (25 mmol =15,3g) wird bei -10°C vorgelegt und Aktivkohle (0,25g) zur beschleunigten Bildung von Phosgen zugegeben. Das in Essigsäureethylester p.a. (20ml) gelöste δ-Valerolactam (25mmol = 2,55g) wird in 1,5 Std. bei -10 bis 0°C zugetropft. Man läßt auf 25°C erwärmen bis im Dünnschichtchromatogramm kein δ-Valerolactam mehr nachzuweisen ist. Es wird langsam auf 50°C erwärmt, wobei eine starke Gasentwicklung zu erkennen ist. Im Dünnschichtchromatogramm erkennt man nur einen Produktfleck. Die Aktivkohle wird abfiltriert und das Filtrat eingeengt. 4,25g ( = 94.4 % Ausbeute ) des Produkts werden als hellgelbes Öl isoliert.

### 2) Ameisensäure N'-(2,4-dichloro-5-hydroxyphenyl)-hydrazid Natriumsalz

Zu einer Lösung von 30 %-iger, methanolischer Lösung von Natriummethanolat (22,5 g, 0,125 mol) in Methanol wird bei Raumtemperatur 2,4-Dichlor-5-hydroxyphenylhydrazin-Hydrochlorid (11,5 g, 0,05 mol) eingetragen. Es bildet sich eine Suspension, zu der bei der gleichen Temperatur Methylformiat (62 ml, 1 mol) zugegeben wird. Anschließend wird 2 Stunden bei 40-50°C und dann über Nacht bei Raumtemperatur gerührt. Das Produkt wird abfiltriert, der Filterrückstand mit Methanol gewaschen und anschließend das Filtrat unter verringertem Druck eingeengt. Der Rückstand wird in Toluol aufgenommen und beim Einengen unter verringertem Druck bildet sich ein hellbraunes Pulver, das mit dem obengenannten Filterrückstand vereinigt wird.
Ausbeute: 12,5 g (= 99 %)
Schmelzpunkt: 202-204°C (unter Zersetzung)

### 3) Ameisensäure N'-(2,4-dichloro-5-(2-propinyloxy)-phenyl)-hydrazid

Eine gerührte Suspension von Ameisensäure N'-(2,4-dichloro-5-hydroxyphenyl)-hydrazid Natriumsalz (1.1 g, 5 mmol) in einem Gemisch von 25 ml Aceton und 25 ml Wasser wird bei Raumtemperatur mit Benzolsulfonsäure-(2-propargyl)ester (1.01 g, 5 mmol) versetzt. Es wird Natronlauge (0,2 g, 5 mmol in 5 ml Wasser) innerhalb von 30 Minuten zugetropft, so daß der pH-Wert zwischen 9,5 und 10 liegt. Man versetzt mit 10 ml Methanol um eine klare Lösung zu erzeugen. Es wird 30 Minuten auf 40°C erwärmt, wobei sich ein Niederschlag bildet. Man rührt über Nacht, filtriert das Produkt ab und wäscht mit Wasser nach.
Ausbeute: 0,8 g (61 %) hellbraune Kristalle
Schmelzpunkt: 184°C

### 4) 2-[2,4-Dichlor-5-(2-propynyloxy)phenyl]-5,6,7,8-tetrahydro-1,2,4-triazolo[4,3-a]pyridin-3(2H)-on

Zu einer gerührten Suspension von Ameisensäure N'-(2,4-dichloro-5-(2-propinyloxy)-phenyl)-hydrazid (5.18 g, 0,02 mol) in 250 ml Acetonitril wird bei 0-10°C in 20 Minuten N-Chlorcarbonyl-2-chlor-1,4,5,6-tetrahydropyridin (4,32 g, 0,024 mol), gelöst in 20 ml Acetonitril gegeben. Anschließend versetzt man bei der gleichen Temperatur mit Triethylamin(4,04 g, 0,04 mol) und gelöst in 20 ml Acetonitril.
Man rührt eine Stunde bei 5-10°C und ein farbloser Niederschlag fällt aus. Man erwärmt 4 Stunden auf 50-55°C und rührt anschließend eine Nacht bei Raumtemperatur. Der Ansatz wird mit 30 ml 6N Salzsäure versetzt und 1 Stunde bei 50°C gerührt. Das Lösungsmittel wird unter verringertem Druck abgezogen und der Rückstand mit Eiswasser digeriert. Der entstehende hellgraue Niederschlag wird durch Filtration abgetrennt, mit Wasser gewaschen und bei 50-60°C getrocknet.
Ausbeute: 6,6 g (= 97,6 %)
Schmelzpunkt: 160-162°C

## Patentansprüche

1. Verfahren zur Herstellung von Verbindungen des Typs (I), 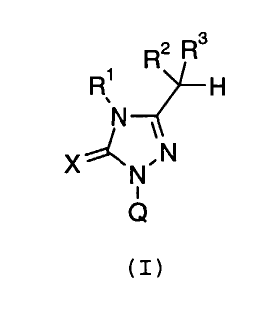 worin
X = O oder S,
R¹ = (C₂-C₈) Alkoxyalkyl, (C₂-C₈) Haloalkoxyalkyl, (C₁-C₈) Alkyl, (C₁-C₈) Haloalkyl, (C₁-C₈) Cyanoalkyl, (C₂-C₈) Alkylthioalkyl, (C₂-C₈) Alkylsulfinylalkyl, (C₂-C₈) Alkylsulfonylalkyl, (C₇-C₈) Arylalkyl wie Benzyl, (C₂-C₈) Alkenyl, (C₂-C₈) Haloalkenyl, (C₂-C₈) Alkinyl, (C₂-C₈) Haloalkinyl, Aryl wie Naphthyl oder Phenyl, das ein- oder mehrfach mit Halogen, (C₁-C₃) Alkyl oder (C₁-C₃) Alkoxy substituiert sein kann, Heteroaryl wie Pyridin, wobei Heteroaryl ggf. mit Halogen substituiert sein kann, bedeuten,
R² und R³, unabhängig voneinander, H, (C₂-C₆) Alkoxyalkyl, (C₂-C₆) Haloalkoxyalkyl, (C₁-C₆) Alkyl, (C₁-C₆) Haloalkyl, (C₁-C₆) Cyanoalkyl, (C₁-C₆) Alkylthio, (C₂-C₆) Alkylthioalkyl, (C₂-C₆) Alkylsulfinylalkyl, (C₂-C₆) Alkylsulfonylalkyl, (C₇-C₈) Arylalkyl wie Benzyl, (C₂-C₆) Alkenyl, (C₂-C₆) Haloalkenyl, (C₂-C₆) Alkinyl, (C₂-C₆) Haloalkinyl, Aryl wie Naphthyl oder Phenyl, das ein- oder mehrfach substituiert sein kann mit Halogen, (C₁-C₃) Alkyl oder (C₁-C₃) Alkoxy, Heteroaryl wie Pyridin, wobei
R¹ und R² auch zu einem Ring verbunden sein können, so daß sich Struktur (II) ergibt, 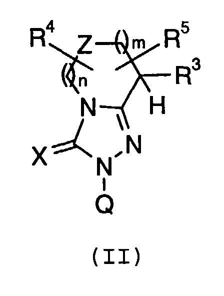 worin
n und m unabhängig voneinander 0, 1, 2 oder 3 sind,
Z = CR⁴R⁵, O, S, S(O), S(O)₂, N((C₁-C₄)Alkyl), N((C₁-C₄)Haloalkyl), C=O, C=N-R⁴ oder C=S,
R⁴ = H, (C₁-C₃) Alkyl, Halogen, (C₁-C₆) Alkoxy, (C₁-C₆) Haloalkyl, (C₁-C₆) Haloalkoxy, (C₂-C₆) Alkylcarbonyloxy oder (C₂-C₆) Haloalkylcarbonyloxy,
R⁵ = H, (C₁-C₃) Alkyl oder Halogen,
wobei R⁴ und R⁵, unabhängig voneinander, ein- oder mehrfach den Ring substituieren können und bis zu 12 (m = 3, n = 3) Positionen einnehmen können,
bedeuten,
Q für einen der Reste 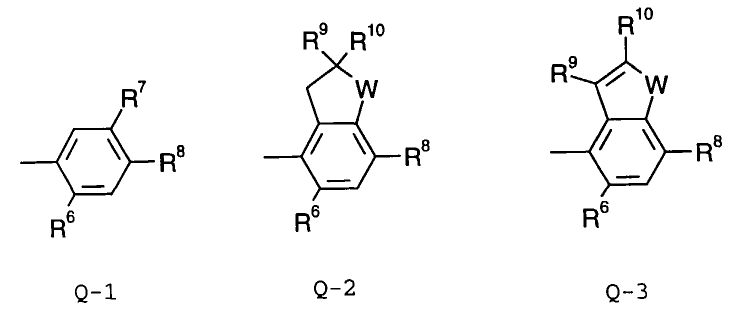 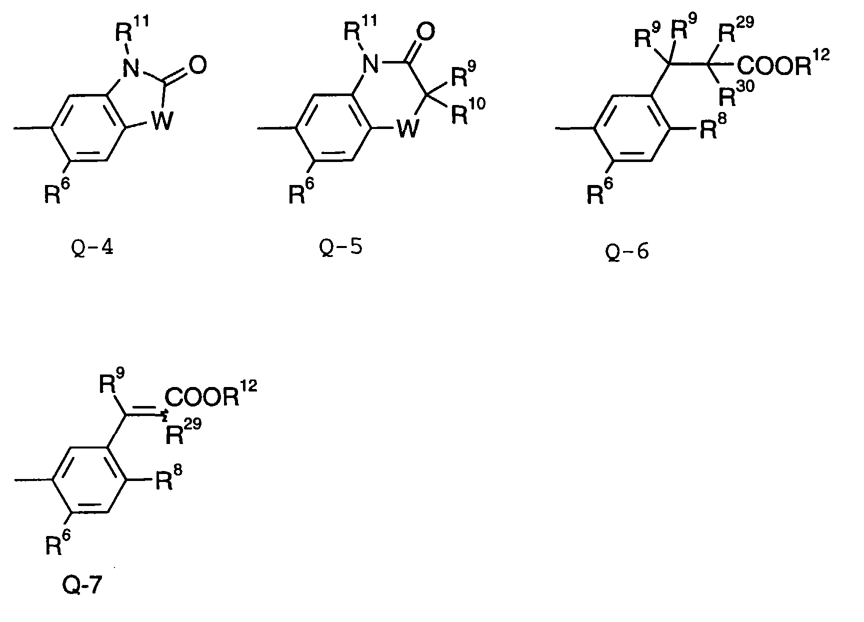 steht, wobei
W = O oder S,
R⁶ = H, Halogen,
R⁷ = H, (C₁-C₈) Alkyl, (C₁-C₈) Haloalkyl, Halogen, OH, OR¹², SH, S(O)ₚR¹², COR¹², CO₂R¹², C(O)SR¹², C(O)NR¹⁴R¹⁵, CHO, CR¹⁴=NOR²¹, CHCR²²CO₂R¹², CH₂CHR²²CO₂R¹², CO₂N=CR¹⁶R¹⁷, NO₂, CN, NHSO₂R¹⁸, NHSO₂NHR¹⁸, NR¹²R²³, NH₂ oder Phenyl, wahlweise substituiert mit R²⁴,
p = 0,1 oder 2,
R⁸ = (C₁-C₂) Alkyl, (C₁-C₂) Haloalkyl, OCH₃, SCH₃, OCHF₂, Halogen, CN oder NO₂,
R⁹ = H, (C₁-C₃) Alkyl oder Halogen,
R¹⁰ = H, (C₁-C₃) Alkyl, Halogen, (C₁-C₃) Haloalkyl, Cyclopropyl, Vinyl, C₂-Alkinyl, CN, C(O)R²³,CO₂R²³, C(O)NR²³R²⁵, CR¹⁹R²⁰CN, CR¹⁹R²⁰C(O)R²³, CR¹⁹R²⁰CO₂R²³, CR¹⁹R²⁰C(O)NR²³R²⁵, CHR¹⁹OH, CHR¹⁹OC(O)R²³ oder OCHR¹⁹OC(O)NR²³R²⁵, oder, wenn Q gleich Q-2 ist, können R⁹ und R¹⁰ zusammen mit dem Kohlenstoffatom, an dem sie gebunden sind, C=O sein,
R¹¹ = H, (C₁-C₆) Alkyl, (C₁-C₆) Haloalkyl, (C₃-C₆) Haloalkenyl, (C₂-C₆) Alkoxyalkyl, (C₃-C₆) Alkenyl, (C₃-C₆) Alkinyl, (C₃-C₆) Haloalkinyl, (C₄-C₇) Cycloalkylalkyl, 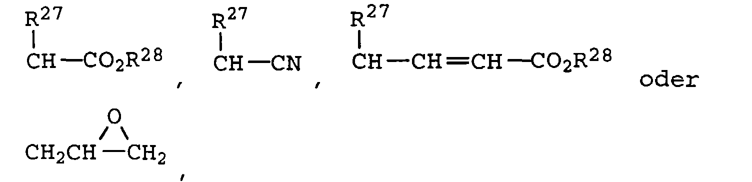
R¹² = (C₁-C₈) Alkyl, (C₃-C₈) Cycloalkyl, (C₃-C₈) Alkenyl, (C₃-C₈) Alkinyl, (C₁-C₈) Haloalkyl, (C₂-C₈) Alkoxyalkyl, (C₂-C₈) Alkylthioalkyl, (C₂-C₈) Alkylsulfinylalkyl, (C₂-C₈) Alkylsulfonylalkyl, (C₄-C₈) Alkoxyalkoxyalkyl, (C₄-C₈) Cycloalkylalkyl, (C₆-C₈) Cycloalkoxyalkyl, (C₄-C₈) Alkenyloxyalkyl, (C₄-C₈) Alkinyloxyalkyl, (C₃-C₈) Haloalkoxyalkyl, (C₄-C₈) Haloalkenyloxyalkyl, (C₄-C₈) Haloalkinyloxyalkyl, (C₆-C₈) Cycloalkylthioalkyl, (C₄-C₈) Alkenylthioalkyl, (C₄-C₈) Alkinylthioalkyl, (C₁-C₄) Alkyl, substituiert mit Phenoxy oder Benzyloxy, jeder Ring wahlweise substituiert mit Halogen, (C₁-C₃) Alkyl oder (C₁-C₃) Haloalkyl, (C₄-C₈) Trialkylsilylalkyl, (C₃-C₈) Cyanoalkyl, (C₃-C₈) Halocycloalkyl, (C₃-C₈) Haloalkenyl, (C₅-C₈) Alkoxyalkenyl, (C₅-C₈) Haloalkoxyalkenyl, (C₅-C₈) Alkylthioalkenyl, (C₃-C₈) Haloalkinyl, (C₅-C₈) Alkoxyalkinyl, (C₅-C₈) Haloalkoxyalkinyl, (C₅-C₈) Alkylthioalkinyl, (C₂-C₈) Alkylcarbonyl, Benzyl, wahlweise substituiert mit Halogen, (C₁-C₃) Alkyl oder (C₁-C₃) Haloalkyl, CHR¹⁹COR¹³, CHR¹⁹P(O)(OR¹³)₂, CHR¹⁹P(S)(OR¹³)₂, P(O)(OR¹³)₂, P(S)(OR¹³)₂, CHR¹⁹C(O)NR¹⁴R¹⁵, CHR¹⁹C(O)NH₂, CHR¹⁹CO₂R¹³, CO₂R¹³, SO₂R¹³, Phenyl, wahlweise substituiert mit R²⁴, 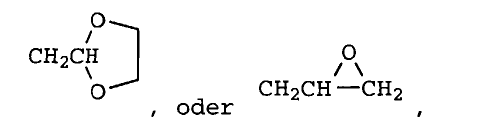
R¹³ = (C₁-C₆) Alkyl, (C₁-C₆) Haloalkyl, (C₃-C₆) Alkenyl oder (C₃-C₆) Alkinyl,
R¹⁴ und R¹⁶ = unabhängig voneinander, H oder (C₁-C₄) Alkyl,
R¹⁵ und R¹⁷ = unabhängig voneinander, (C₁-C₄) Alkyl oder Phenyl, wahlweise substituiert mit Halogen, (C₁-C₃) Alkyl oder (C₁-C₃) Haloalkyl, oder
R¹⁴ und R¹⁵ können zusammen mit dem Stickstoffatom, das sie verbindet, einen Piperidinyl-, Pyrrolidinyl- oder Morpholinyl-Ring bilden, jeder Ring wahlweise substituiert mit (C₁-C₃) Alkyl, Phenyl oder Benzyl, oder
R¹⁶ und R¹⁷ können zusammen mit dem Kohlenstoffatom, das sie verbindet, (C₃-C₈) Cycloalkyl sein,
R¹⁸ = (C₁-C₄) Alkyl oder (C₁-C₄) Haloalkyl,
R¹⁹ und R²⁰ = unabhängig H oder (C₁-C₅) Alkyl,
R²¹ = H, (C₁-C₆) Alkyl, (C₃-C₆) Alkenyl oder (C₃-C₆) Alkinyl,
R²² und R²⁷ = unabhängig voneinander, H, (C₁-C₄) Alkyl oder Halogen, oder
R¹² und R²² können zusammen ein (C₂-C₃) Alkylen bilden,
R²³, R²⁴ und R²⁸ = unabhängig voneinander, H oder (C₁-C₄) Alkyl,
R²⁵ = (C₁-C₂) Alkyl, (C₁-C₂) Haloalkyl, OCH₃, SCH₃, OCHF₂, Halogen, CN oder NO₂ und
R²⁶ = H, (C₁-C₅) Alkyl, (C₁-C₅) Haloalkyl, (C₃-C₆) Cycloalkyl, (C₃-C₆) Halocycloalkyl, (C₁-C₅) Alkoxy, (C₁-C₅) Haloalkoxy und Phenyl, das ggf. bis zu dreifach wahlweise mit Halogen, NO₂, Cyano, (C₁-C₂) Alkyl, (C₁-C₂) Haloalkyl, (C₁-C₂) Alkoxy oder (C₁-C₂) Haloalkoxy substituiert sein kann,
R²⁹ = H, Halogen oder (C₁-C₆) Alkyl,
R³⁰ = H, (C₁-C₄) Alkyl oder Halogen,
bedeutet,
**dadurch gekennzeichnet**,
daß Amide der allgemeinen Formel (IV) 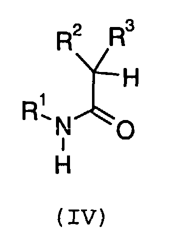 worin R¹, R² und R³ die oben angegebene Bedeutung besitzen, in an sich bekannter Weise mit Phosgen bzw. Thiophosgen oder einem Phosgenersatz bzw. Thiophosgenersatz wahlweise in einem Lösungsmittel zu einer Verbindung der Formel (V) 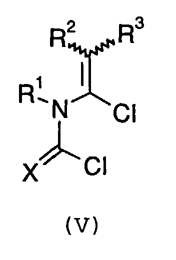 umgesetzt werden, worin R¹, R², R³ und X die oben angegebene Bedeutung besitzen, und diese Verbindungen der Formel (V) wird anschließend mit einer Verbindung der Formel (VI) oder ein Säureadditionssalz hiervon,
Q―NH-NH-CO-R²⁶ (VI)
worin Q und R²⁶ die oben angegebene Bedeutung besitzt, wahlweise in Gegenwart eines Verdünnungsmittels und wahlweise zunächst in Gegenwart einer Base und anschließend in Gegenwart einer Säure und wahlweise durch Temperaturerhöhung zu einer Verbindung der Formeln (I) oder (II) umsetzt.

2. Verfahren zur Herstellung von Verbindungen des Typs II, 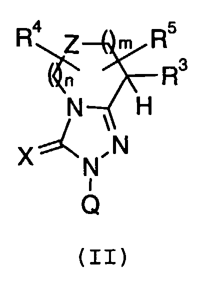 wobei
X = 0,
p = 0,1,2,3
n und m unabhängig voneinander 0, 1 oder 2 sind,
Z = CR⁴R⁵, O, S, C=O oder C=S,
R⁴ = H, (C₁-C₂) Alkyl, (C₁-C₂) Haloalkyl, Fluor, Chlor, (C₁-C₃) Alkoxy oder (C₁-C₃) Haloalkoxy,
R³ und R⁵ können unabhängig voneinander H, (C₁-C₂) Alkyl, Fluor oder Chlor sein,
Q = Q-1, Q-2 oder Q-5,
R⁷ = H, (C₁-C₈) Alkyl, (C₁-C₈) Haloalkyl, Halogen, OH, OR¹², SH, S(O)ₚR¹², COR¹², CO₂R¹², C(O)SR¹², C(O)NR¹⁴R¹⁵, CHO, CH=CHCO₂R¹², CO₂N=CR¹⁶R¹⁷, NO₂, CN, NHSO₂R¹⁸ oder NHSO₂NHR¹⁵ und
R¹² = (C₁-C₄) Alkyl, (C₃-C₄) Alkenyl, (C₃-C₄) Alkinyl, (C₂-C₄) Alkoxyalkyl, (C₁-C₄) Haloalkyl, (C₃-C₄) Haloalkenyl oder (C₃-C₄) Haloalkinyl,
R²⁶ = H
bedeutet,
**dadurch gekennzeichnet**,
daß man Amide der allgemeinen Formel VII 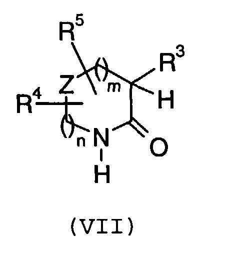 worin n, m, Z, R³, R⁴ und R⁵ die oben angegebene Bedeutung haben, in an sich bekannter Weise mit Phosgen bzw. Thiophosgen oder einem Phosgenersatz bzw. Thiophosgenersatz wahlweise in einem Lösungsmittel zu einer Verbindung der Formel VIII 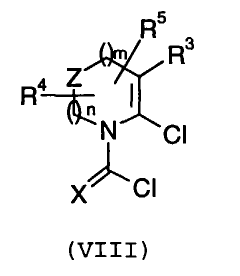 umsetzt, worin n, m, X, Z, R³, R⁴ und R⁵ die oben angegebene Bedeutung besitzen und diese Verbindung der Formel (VIII) anschließend mit einer Verbindung der Formel (VI) oder eines Säureadditionssalzes hiervon,
Q―NH-NH-CO-R²⁶ (VI)
worin Q und R²⁶ die oben angegebene Bedeutung haben, wahlweise in Gegenwart eines Verdünnungsmittels und wahlweise zunächst in Gegenwart einer Base und anschließend in Gegenwart einer Säure und wahlweise durch Temperaturerhöhung zu einer Verbindung der Formel (II) umsetzt.

3. Verfahren zur Herstellung von Verbindungen des Typs II, 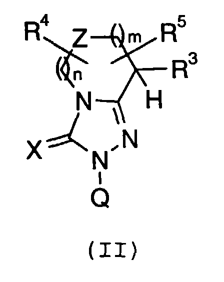 worin
X = 0,
n und m unabhängig voneinander 0, 1 oder 2 sind,
Z = CH₂, CHF, CF₂ oder CHCl,
R⁴ = H, (C₁-C₂) Alkyl, (C₁-C₂) Haloalkyl, Fluor, Chlor ist,
R⁵ = H, (C₁-C₂) Alkyl, Fluor, Chlor ist,
R³ = H ist,
Q = Q-1 oder Q-5 ist,
R⁷ = H, (C₁-C₈) Alkyl, (C₁-C₈) Haloalkyl, Halogen, OH, OR¹², SH, S(O)ₚR¹², COR¹², CO₂R¹², C(O)SR¹², C(O)NR¹⁴R¹⁵, CHO, CH=CHCO₂R¹², CO₂N=CR¹⁶R¹⁷, NO₂, CN, NHSO₂R¹⁸ oder NHSO₂NHR¹⁵ und
R¹² = (C₁-C₄) Alkyl, (C₃-C₄) Alkenyl, (C₃-C₄) Alkinyl, (C₂-C₄) Alkoxyalkyl, (C₁-C₄) Haloalkyl, (C₃-C₄) Haloalkenyl oder (C₃-C₄) Haloalkinyl bedeuten, und
R⁸ = Halogen, CN oder NO₂
R²⁶ = H
bedeuten,
**dadurch gekennzeichnet**,
daß Amide der allgemeinen Formel (VII), 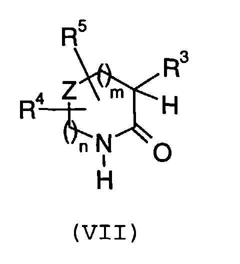 worin n, m, X, Z, R³, R⁴ und R⁵ die oben angegebene Bedeutung haben, in an sich bekannter Weise mit Phosgen bzw.Thiophosgen oder Phosgenersatz bzw. Thiophosgenersatz zu Verbindungen des Typs (VIII) 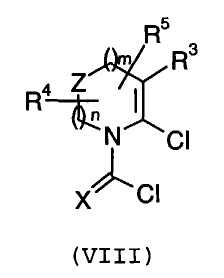 umgesetzt werden und diese Verbindung des Typs (VIII) anschließend mit einer Verbindung der Formel (VI) oder eines Säureadditionssalzes hiervon
Q―NH-NH-CO-R²⁶ (VI)
worin Q und R²⁶ die oben angegebene Bedeutung haben, wahlweise in Gegenwart eines Verdünnungsmittels und wahlweise zunächst in Gegenwart einer Base und anschließend in Gegenwart einer Säure und wahlweise durch Temperaturerhöhung zu einer Verbindung der Formel (II) umsetzt.

4. Verfahren zur Herstellung der Verbindung (IX) 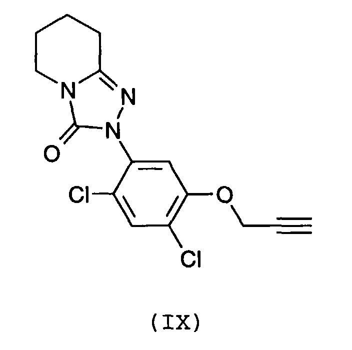 **dadurch gekennzeichnet**,
daß man 2-Piperidon mit Phosgen oder einem Phosgenersatz wahlweise in einem Lösungsmittel in an sich bekannter Weise zu einer Verbindung der Formel (X) 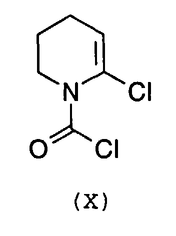 umsetzt, und diese Verbindung der Formel (X)
anschließend mit einer Verbindung der Formel (XI) 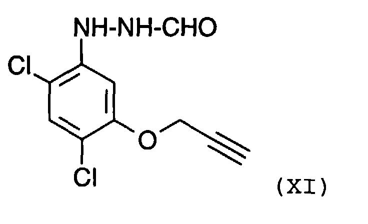 wahlweise in Gegenwart eines Verdünnungsmittels und wahlweise in Gegenwart einer Base und anschließend in Gegenwart einer Säure und wahlweise durch Temperaturerhöhung zu einer Verbindung der Formel (IX) reagieren läßt.

5. Verfahren nach einem der vorhergehenden Ansprüche,
**dadurch gekennzeichnet**,
daß die Synthesefolge ausgehend von den Verbindungen der allgemeinen Formeln (X) und (XI) ohne Zwischenisolierung in einem Reaktionsgefäß erfolgen.

6. Verfahren nach einem der vorhergehenden Ansprüche,
**dadurch gekennzeichnet**,
daß die genannte Synthesefolge ohne Wechsel des Lösungsmittels erfolgen.

7. Verfahren nach einem der vorhergehenden Ansprüche,
**dadurch gekennzeichnet**,
daß als Säure organische oder anorganische Säuren wie beispielsweise HCl, H₂SO₄ HNO₃, HCO₂H, *p*-Toluolsulfonsäure, Essigsäure, Camphersulfonsäure oder H₃PO₄ oder eine saurer Ionenaustauschers verwendet werden.

8. Verfahren nach Anspruch 7,
**dadurch gekennzeichnet**,
daß als Säure Chlorwasserstoff, Schwefelsäure oder Phosphorsäure verwendet wird.

9. Verfahren nach einem der vorhergehenden Ansprüche,
**dadurch gekennzeichnet**,
daß die Reaktionsfolge bei einer Temperatur zwischen -20 °C und 120 °C, vorzugsweise zwischen -20 °C und 80 °C, durchgeführt wird.

10. Verfahren nach einem der vorhergehenden Ansprüche,
**dadurch gekennzeichnet**,
daß als Lösungsmittel Essigsäureethylester oder Acetonitril oder Gemische der beiden verwendet werden.

11. Verfahren nach einem der vorhergehenden Ansprüche,
**dadurch gekennzeichnet**,
daß als Base eine organische Base, eine anorganische Base oder ein Alkoholat verwendet wird.

12. Verfahren nach Anspruch 11,
**dadurch gekennzeichnet**,
daß als Base vorzugsweise Triethylamin, Tributylamin, Pyridin, Tetramethylpiperidin, NaOH, KOH, Ca(OH)₂, Na₂CO₃, K₂CO₃, CaCO₃, NaHCO₃, KHCO₃, NaOCH₃ oder NaOC₂H₅ verwendet wird.

13. Verfahren nach Anspruch 12,
**dadurch gekennzeichnet**,
daß als Base besonders bevorzugt K₂CO₃, Na₂CO₃, Triethylamin oder Tributylamin verwendet werden.

14. Ameisensäure N'-(2,4-dichloro-5-hydroxy-phenyl)-hydrazid (XIV)

15. Ameisensäure N'-[2,4-dichloro-5-(prop-2-inyloxy)-phenyl]-hydrazid (XI)
